Europäisches Patentamt

European Patent Office

Office européen des brevets

(11) Publication number: **0 289 690**
**A1**

## EUROPEAN PATENT APPLICATION

(21) Application number: **87830278.5**

(22) Date of filing: **17.07.87**

(51) Int. Cl.4: **A44B 15/00 , A61F 5/43**

(30) Priority: **04.05.87 IT 2150987 U**

(43) Date of publication of application:
**09.11.88 Bulletin 88/45**

(84) Designated Contracting States:
**AT BE CH DE ES FR GB GR LI LU NL SE**

(71) Applicant: **FRAMIS S.r.l.**
**Via Andrea Doria, 17**
**I-20124 Milano(IT)**

(72) Inventor: **Salotto, Luciano FRAMIS S.r.l.**
**Via Andrea Doria, 17**
**I-20124 Milano(IT)**

(74) Representative: **Cicogna, Franco**
**Ufficio Internazionale Brevetti Dott.Prof.**
**Franco Cicogna Via Visconti di Modrone,**
**14/A**
**I-20122 Milano(IT)**

(54) **A key-ring provided with a pendant in the form of an openable container.**

(57) A key-ring for promotional and publicity use as a loop (1) for receiving keys joined by a short length of chain (2) to a pendant (3) in the form of a flat closable box for housing a condom (4). The pendant (3) is defined by two opposite flat parallel walls (5,6) one of which (6) is transparent or transluscent and formed as an openable closure through which the presence of the condom in the container can be observed, and the other of which (5) is formed with a flat area for receiving writing or drawings advising on its use.

FIG. 1

EP 0 289 690 A1

## "A key-ring provided with a pendant in the form of an openable container"

The present invention relates to a key-ring provided with an pendant in the form of an openable container adapted to contain a specific product.

A very wide range of key-rings are already available in varied shapes and formed in different materials. In general key-rings are used as promotional objects in the publicity field. Such key-rings, as well as their obvious function of carrying a certain number of keys, also act to carry, on suitably shaped parts of their surfaces, both decorative motifs, and writing, drawings, symbols or marks, usually for advertisement purposes.

For an optimum yield from the object as a publicity gift, it must naturally not only be useful and easy to use, but also respond to topical criteria.

The key-ring of the present invention tends to respond in a convenient and positive way to the above-mentioned diverse considerations. According to the present invention there is provided a key-ring of the type comprising a closed loop for receiving keys and a pendant secured to the loop, characterised in that the pendant is formed as a closable container shaped and dimensioned to adapt it to receive a condom.

With specific reference to the avoidable risk of contracting various unfortunately diffuse and easily transmissible infections the pendant, in the form of an openable container, has been particularly designed for housing a particular sanitary protection, namely a condom, and also to carry a written warning encouraging its use.

This pendant, which can be made in any material, but preferably in plastics material, preferably has at least one transparent zone, which leaves the enclosed object at least partly exposed to view. The container may have, in practice, any shape as long as it has an appropriate interior space and is openable for the removal of the condom contained therein.

Naturally, for practical and aesthetic motives, the pendant may be linked to the key-ring by a short chain.

One embodiment of the present invention will now be more particularly described, by way of example, with reference to the accompanying drawings, in which:

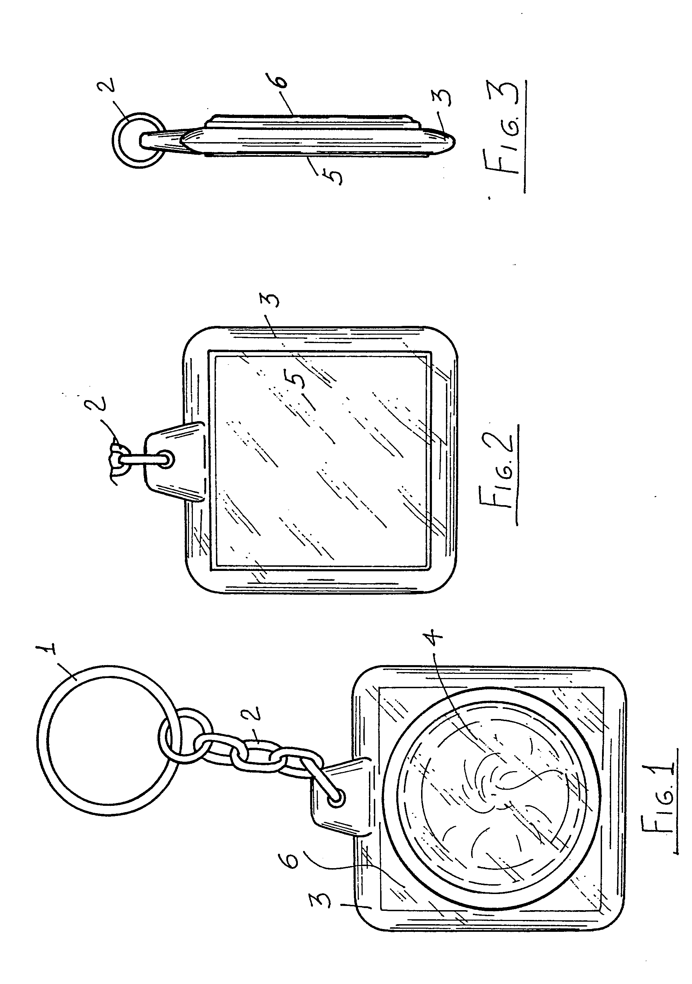

Figure 1 is a front view of the key-ring of the invention seen in the direction of the face of its pendant, which leaves the condom enclosed in it in view;

Figure 2 shows the rear face of the pendant which may carry words and/or various written matter or ideograms or drawings; and

Figure 3 illustrates the pendant seen in side view.

Referring now to the drawings, it can be seen that the key-ring in question comprises a ring 1 of usual type for keys, having a break (not shown) to allow the insertion and removal of keys. Attached to this ring 1 by a short length of chain 2 is a pendant 3 in the form of a flat box-like container enclosing a small interior volume which can contain one or more condoms. The container 3 has a transparent front face 6 through which the presence of a condom 4 in the container can be observed. On the rear face 5 of the pendant there is a wide flat area susceptible of receiving possible wording and/or drawings or various symbols or ideograms.

## Claims

1. A key-ring of the type comprising a closed loop (1) for receiving keys and a pendant (3) secured to the loop (1), characterised in that the pendant (3) is formed as a closable container shaped and dimensioned to adapt it to receive a condom (4).

2. A key-ring according to Claim 1, characterised in that the pendant (3) is linked to the closed loop by means of a short chain (2).

3. A key-ring according to Claim 1 or Claim 2, characterised in that the pendant (3) is formed as a substantially flat box-like container enclosing an interior volume between generally parallel front and rear walls (5,6).

4. A key-ring according to Claim 3, characterised in that one of the flat walls (5,6) of the box-like container constituting the pendant (3) is formed as a closure element (6) for closing the said volume and is transparent or semi-transparent so as to leave the condom (4) contained in the pendant (3) in view when the container is closed.

5. A key-ring according to any preceding Claim, characterised in that the box-like container constituting the pendant (3) has a square outline with rounded corners.

6. A key-ring according to Claim 4 or Claim 5, characterised in that the other of the flat walls (5) of the box-like container constituting the pendant (3) has a face adapted to receive wording and/or drawings and/or symbols relating to the condom contained therein.

Fig. 3

Fig. 2

Fig. 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| E | US-A-4 741 434 (LIEBMAN)<br>* Column 1, line 29 - column 2, line 26 *<br>--- | 1-6 | A 44 B 15/00<br>A 61 F 5/43 |
| E | DE-U-8 703 111 (VOLKER)<br>* Pages 2-4 *<br>--- | 1 | |
| A | DE-C- 441 927 (EISINGER)<br>* Pages 1,2 *<br>--- | 1 | |
| A | FR-A-1 488 397 (BARREE)<br>* Page 1, column 1, line 33 - column 2, line 20 *<br>--- | 1-6 | |
| A | DE-A-2 538 117 (MEYER)<br>* Page 6, line 30 - page 7, line 23 *<br>--- | 1-6 | |
| A | US-A-3 111 152 (GOESSLING)<br>* Column 1, line 1 - column 4, line 33 *<br>----- | 1,3-6 | |

TECHNICAL FIELDS SEARCHED (Int. Cl.4)

A 44 B
A 61 F

The present search report has been drawn up for all claims

| Place of search | Date of completion of the search | Examiner |
|---|---|---|
| THE HAGUE | 05-08-1988 | VAN OORSCHOT J.W.M. |

EPO FORM 1503 03.82 (P0401)